# EUROPÄISCHE PATENTANMELDUNG

(11) **EP 2 224 225 A1**
(43) Veröffentlichungstag der Anmeldung: **01.09.2010**
(21) Anmeldenummer: 09002843.2
(22) Anmeldetag: 27.02.2009
(51) Int. Cl.: G01N 21/03, A61B 18/20, G01N 21/71

(54) **Einrichtung zur Untersuchung einer Probe durch Erzeugung eines Temperatursprungs in der Probe**

(71) Anmelder: Medizinische Hochschule Hannover, 30625 Hannover (DE)
(72) Erfinder: Manstein, Dietmar, 30559 Hannover (DE); Chizhov, Igor, 30171 Hannover (DE)
(74) Vertreter: Wagner, Carsten

(57) **Zusammenfassung**

Eine Einrichtung 2 zur Untersuchung einer Probe 4 durch Erzeugung eines Temperatursprungs in der Probe 4, weist eine Aufnahme 6 für die Probe 4 und Mittel zur Erzeugung eines Temperatursprungs in der Probe 4 zur Erreichung einer Untersuchungstemperatur auf, die einen ersten Laser 12 aufweisen. Die Einrichtung 2 weist ferner Mittel zur Aufrechterhaltung der Untersuchungstemperatur auf, die wenigstens einen zweiten Laser 18 aufweisen.

## Beschreibung

Die Erfindung betrifft eine Einrichtung der im Oberbegriff des Anspruchs 1 genannten Art zur Untersuchung einer Probe durch Erzeugung eines Temperatursprungs in der Probe.

Einrichtungen der betreffenden Art sind bekannt und werden zur Untersuchung von Proben in Bezug auf biologische, chemische oder materialwissenschaftliche Reaktionen im Rahmen der sogenannten "Temperature Jump"(T-Jump)-Spektroskopie verwendet. Bei diesem Verfahren wird in der Probe ein Temperatursprung erzeugt, wobei durch die Temperaturänderung verursachte Gleichgewichtsstörungen, Neuverteilungen oder Konfirmationsänderungen spektroskopisch untersucht werden. Bei den Proben kann es sich insbesondere um Proteine handeln.

Die bekannten Einrichtungen weisen eine Aufnahme für die Probe sowie Mittel zur Erzeugung eines Temperatursprungs in der Probe zur Erreichung einer Untersuchungstemperatur auf. Bei den bekannten Einrichtungen wird der Temperatursprung durch Wärmeeintrag in die Probe durch Mikrowellen oder eine kapazitive Entladung erzeugt. Ein Nachteil der bekannten Einrichtung besteht darin, daß die Temperatur in der Probe nach Erreichung der Untersuchungstemperatur relativ schnell sinkt, so daß der Zeitraum, innerhalb dessen die Probe nach dem Temperatursprung untersucht werden kann, relativ gering ist und beispielsweise von wenigen Mikrosekunden bis etwa 20 bis 50 ms reicht.

Der Erfindung liegt die Aufgabe zugrunde, eine Einrichtung der im Oberbegriff des Anspruchs 1 genannten Art zur Untersuchung einer Probe durch Erzeugung eines Temperatursprungs in der Probe anzugeben, die den Nachteil der bekannten Einrichtungen nicht aufweist, bei der also die Zeitdauer, innerhalb derer nach der Erzeugung des Temperatursprungs Untersuchungen der Probe durchgeführt werden können, verlängert ist.

Diese Aufgabe wird durch die im Anspruch 1 angegebene Erfindung gelöst.

Erfindungsgemäß ist vorgesehen, daß der zur Erzielung des Temperatursprungs erforderliche Energieeintrag in die Probe über einen Laser vollzogen wird. Dementsprechend sieht die Erfindung vor, daß die Mittel zur Erzeugung eines Temperatursprungs in der Probe zur Erreichung einer Untersuchungstemperatur einen ersten Laser aufweisen. Weiterhin sieht die Erfindung vor, daß Mittel zur Aufrechterhaltung der Untersuchungstemperatur vorgesehen sind, die wenigstens einen zweiten Laser aufweisen. Beispielsweise und insbesondere kann es sich bei dem ersten Laser und/oder dem zweiten Laser um einen Infrarot-Laser handeln.

Erfindungsgemäß wird mittels des ersten Lasers, bei dem es sich beispielsweise und insbesondere um einen gepulsten Raman-Laser mit einer Wellenlänge von 1,9 µm und einer Leistung von 100 mJ handeln kann, ein Temperatursprung in der Probe erzeugt. Eine Dissipation der von diesem ursprünglichen Temperatursprung herrührenden Wärme beginnt nach etwa 10 ms im wesentlichen Maße aufgrund von Wärmeleitung zwischen der Probe und der Aufnahme, bei der es sich beispielsweise und insbesondere um eine Untersuchungskammer handeln kann. Um die Temperatur in der Probe konstant und/oder im wesentlichen konstant zu halten, wird der zweite Laser verwendet, bei dem es sich beispielsweise und insbesondere um einen kontinuierlich emittierenden Thulium-Faser-Laser mit einer Wellenlänge von 1,94 µm und 15 W handeln kann, der kontinuierlich emittiert. Durch Kombiantion der beiden Laser ist es erfindungsgemäß möglich, eine vorgegebene Untersuchungstemperatur über einen Zeitraum von 10 s oder länger aufrechtzuerhalten. Auf diese Weise sind die Möglichkeiten zur Untersuchung der Probe wesentlich erweitert, so daß auch der Anwendungsbereich von T-Jump-Systemen wesentlich erweitert ist.

Die erfindungsgemäße Einrichtung ist besonders gut im Bereich der Untersuchung von Proteinen, der Biotechnologie, der Materialwissenschaften, der Nanotechnologie und der Chemie verwendbar.

Ein weiterer Vorteil der erfindungsgemäßen Einrichtung besteht darin, daß zur Durchführung von Untersuchungen ein Probenvolumen ausreichend ist, das wesentlich geringer ist als bei bekannten Verfahren.

Eine weitere Lösung der der Erfindung zugrundeliegenden Aufgabe ist im Anspruch 2 angegeben. Bei dieser Konfiguration weisen die Mittel zur Erzeugung eines Temperatursprungs in der Probe einen ersten Laser und einen zweiten Laser auf, wobei Steuerungsmittel zur wahlweisen Ansteuerung der Laser vorgesehen sind. Bei dieser Ausführungsform kann zur Erzeugung eines Energieeintrags in die Probe entweder der erste Laser oder der zweite Laser oder beide Laser in Kombination miteinander herangezogen werden, wobei die Ansteuerung der Laser hinsichtlich ihrer Leistung und des Zeitpunktes bzw. der Zeitdauer, während der die Laser emittieren, innerhalb weiter Grenzen wählbar ist.

Vorteilhafte Weiterbildungen der Erfindung sehen vor, daß der erste Laser gepulste Laserstrahlung emittiert und/oder daß der zweite Laser kontinuierliche Laserstrahlung emittiert. Auf diese Weise ergeben sich hinsichtlich der Erzeugung des Temperatursprunges und der Aufrechterhaltung der Untersuchungstemperatur besonders günstige Verhältnisse.

Eine andere Weiterbildung der Erfindung sieht vor, daß der erste Laser und der zweite Laser ein Infrarot-Laser sind bzw. ist. Derartige Laser sind besonders gut für den gewünschten Verwendungszweck, nämlich die Erzeugung eines Temperatursprunges und die Aufrechterhaltung einer durch den Temperatursprung erreichten Untersuchungstemperatur einer in einer wässrigen Lösung befindlichen Probe geeignet.

Besonders günstige Verhältnisse hinsichtlich der Erzeugung des Temperatursprunges bzw. der Aufrechterhaltung der Untersuchungstemperatur ergeben sich dann, wenn der erste Laser ein Nd:YAG-Laser bzw. der zweite Laser ein Thulium-Faser-Laser ist, wie dies andere bevorzugte Ausführungsformen der Erfindung ergeben.

Eine besonders vorteilhafte Weiterbildung der Erfindung sieht vor, daß dem ersten Laser ein Frequenzwandler nachgeordnet ist, der vorzugsweise ein Raman-Konverter ist. Auf diese Weise ergeben sich hinsichtlich des Energieverteilungsprofiles besonders günstige Verhältnisse.

Eine andere vorteilhafte Weiterbildung der Erfindung sieht vor, daß die Mittel zur Erzeugung eines Temperatursprungs in der Probe und/oder die Mittel zur Aufrechterhaltung der Untersuchungstemperatur durch Steuerungs- und/oder Regelungsmittel steuerbar bzw. regelbar sind. Bei dieser Ausführungsform dienen die Steuerungs- bzw. Regelungsmittel dazu, in flexibler Weise die Parameter des Temperatursprunges zu steuern bzw. zu regeln.

Um bei der vorgenannten Ausführungsform die Steuerungs- und/oder Regelungsmittel besonders einfach und kostengünstig auszugestalten, sieht eine Weiterbildung der vorgenannten Ausführungsform vor, daß die Steuerungs- und/oder Regelungsmittel Datenverarbeitungsmittel aufweisen.

Um eine Kalibrierung der Einrichtung hinsichtlich der durch den Eintrag von Laserenergie erzielten Temperatur zu vereinfachen, sieht eine andere Weiterbildung vor, daß die Steuerungs- und/oder Regelungsmittel eine Detektoreinheit zur Detektion der Probentemperatur aufweisen.

Die Erfindung wird nachfolgend anhand der beigefügten Zeichchnung näher erläutert, in der ein Ausführungsbeispiel einer erfindungsgemäßen Einrichtung zur Untersuchung einer Probe durch Erzeugung eines Temperatursprunges in der Probe dargestellt ist. Dabei bilden alle beanspruchten, beschriebenen und in der Zeichnung dargestellten Merkmale für sich genommen sowie in beliebiger Kombination miteinander den Gegenstand der Erfindung, unabhängig von ihrer Zusammenfassung in den Patentansprüchen und deren Rückbeziehung sowie unabhängig von ihrer Beschreibung bzw. Darstellung in der Zeichnung.

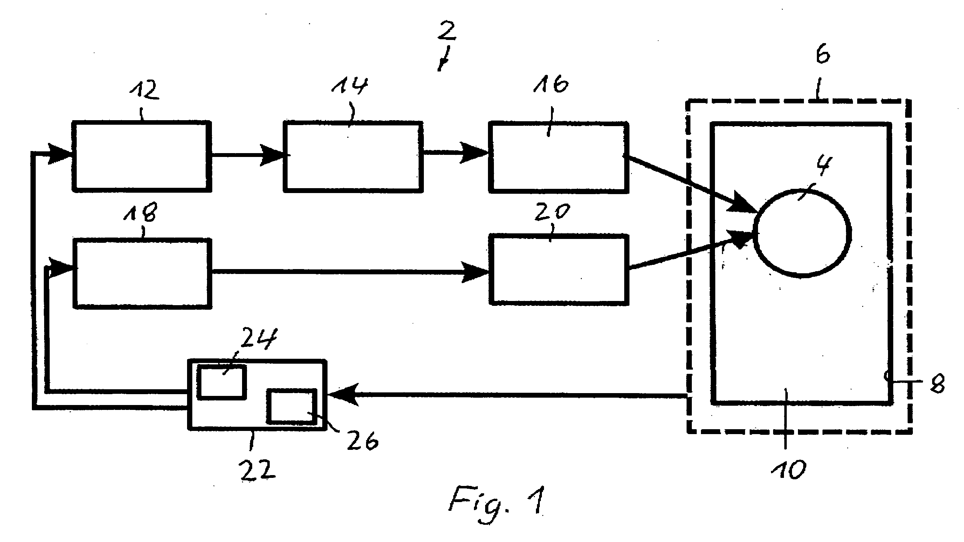

Die Fig. 1 zeigt ein stark schematisiertes Blockschaltbild eines Ausführungsbeispieles einer erfindungsgemäßen Einrichtung 2 zur Untersuchung einer Probe durch Erzeugen eines Temperatursprunges in der Probe. Bei dem dargestellten Ausführungsbeispiel liegt die Probe in einer wässrigen Lösung vor und ist in der Zeichnung symbolisch bei dem Bezugszeichen 4 dargestellt. Die Einrichtung 2 weist eine Aufnahme 6 für die Probe 4 auf, die bei diesem Ausführungsbeispiel eine durch Wandungen 8 aus Quarzglas begrenzte Untersuchungskammer 10 aufweist.

Die Einrichtung 2 weist ferner Mittel zur Erzeugung eines Temperatursprunges in der Probe 4 zur Erreichung einer Untersuchungstemperatur auf, die einen ersten Laser 12 aufweisen, bei dem es sich bei diesem Ausführungsbeispiel um einen Infrarot-Laser, nämlich einen Nd-YAG-Laser, handelt, der bei Benutzung der Einrichtung 2 einen Laserpuls von 5 ns Dauer erzeugt, der in dem Probenvolumen, das bei diesem Ausführungsbeispiel etwa 2 µl beträgt, einen Temperaturvorsprung auf die Untersuchungstemperatur erzeugt. Dem ersten Laser 12 ist ein Frequenzwandler nachgeordnet, der bei diesem Ausführungsbeispiel durch einen Raman-Konverter gebildet ist, der von dem ersten Laser 12 emittierte Laserstrahlung einer Frequenzwandlung unterzieht. Der erste Laser 12 und der Raman-Konverter 14 bilden zusammen einen Raman-Laser mit einer Wellenlänge von 1,9 µm und einer Energie von ca. 100 mJ, um einen Temperaturanstieg von 10°C in der Probe 4 zu erzeugen. Dem Raman-Koverter 14 ist ein Strahlformungsmittel 16 nachgeordnet, das zur Strahlformung des von dem Raman-Laser 12, 14 erzeugten Laserstrahles dient.

Die erfindungsgemäße Einrichtung 2 weist ferner Mittel zur Aufrechterhaltung der Untersuchungstemperatur auf, die bei diesem Ausführungsbeispiel einen zweiten Laser 18 aufweisen. Bei dem dargestellten Ausführungsbeispiel ist der zweite Laser 18 durch einen zweiten Infrarot-Laser in Form eines kontinuierlich emittierenden Thulium-Faser-Lasers gebildet, der bei einer Wellenlänge von 1,94 µm eine Leistung von bis zu 15 W erzeugt. Dem zweiten Laser 18 nachgeordnet ist ein Strahlformungsmittel 20, das den von dem zweiten Laser 18 emittierten Laserstrahl formt. Wie aus Fig. 1 ersichtlich ist, strahlen die Laser 12, 18 in die Untersuchungskammer 10 ein.

Zur Ansteuerung der Laser 12, 18 sind Steuerungsmittel 22 vorgesehen, die bei diesem Ausführungsbeispiel elektronische Datenverarbeitungsmittel 24 aufweisen. Die Steuerungsmittel 22 weisen ferner bei diesem Ausführungsbeispiel eine Detektionseinheit 26 zur Detektion der Probentemperatur auf. Die Detektionseinheit 26 wird beispielsweise verwendet, um die Einrichtung 2 hinsichtlich des bei Einstrahlung von Laserenergie in die Probe 4 erzeugten Temperatursprunges zu kalibrieren.

Die Untersuchung der Probe erfolgt mittels Verfahren der Spektroskopie, die dem Fachmann allgemein bekannt sind und daher hier nicht näher erläutert werden. Dementsprechend sind die Bauteile der zur Durchführung des jeweiligen Spektroskopieverfahrens erforderlichen Einrichtung in der Zeichnung nicht dargestellt.

Die Funktionsweise der erfindungsgemäßen Einrichtung 2 ist wie folgt:

Zur Untersuchung einer Probe wird zunächst der erste Laser 12 angesteuert, der daraufhin einen Laserpuls mit einer Pulsbreite von 5 ns erzeugt, der bei einer Wellenlänge von 1,9 µm und einer Energie von ca. 100 mJ in dem Probenvolumen von 2 µl einen Temperatursprung von etwa 10° erzeugt. Auf diese Weise wird in der Probe eine Untersuchungstemperatur erreicht, bei der die Probe untersucht wird.

Daran anschließend wird mit zeitlicher Verzögerung der zweite Laser 18 angesteuert, so daß dieser bei einer Wellenlänge von 1,94 µm und einer Leistung von bis zu 15 W in die Probe 4 einstrahlt. Die Leistung des zweiten Lasers 18 ist ausreichend, um die Probe 4 innerhalb eines Zeitraumes von weniger als 100 ms auf 100°C zu erhöhen. Erfindungsgemäß wird der zweite Laser 18 hinsichtlich seiner Leistung und des Zeitpunktes und der Zeitdauer der Einstrahlung von Laserenergie in die Probe 4 so angesteuert, daß die Untersuchungstemperatur, die durch den Temperatursprung erreicht wurde, über einen Zeitraum von bis zu 10 s oder länger konstant bleibt. Auf diese Weise sind die Möglichkeiten zur Untersuchung der Probe wesentlich erweitert.

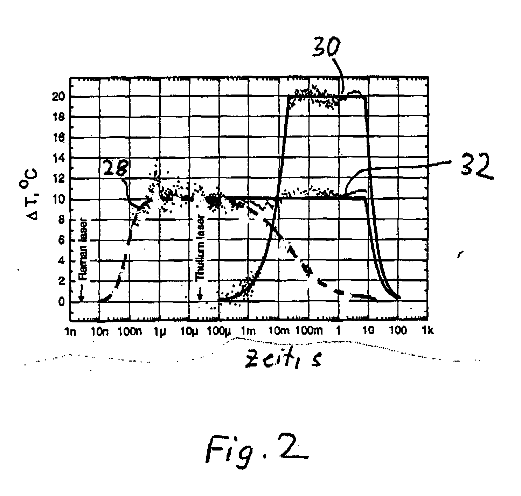

Fig. 2 zeigt den zeitlichen Verlauf der Probentemperatur, wobei die Zeit logarithmisch aufgetragen ist. In Fig. 2 ist durch eine gestrichelte Linie 28 ein zeitlicher Temperaturverlauf dargestellt, der sich ergeben würde, wenn die Probe ausschließlich mittels des ersten Lasers 12 in Verbindung mit dem Raman-Konverter 14 bestrahlt würde. Durch eine erste durchgezogene Linie 30 ist in Fig. 2 ein experimentell ermittelter zeitlicher Temperaturverlauf dargestellt, der sich ergeben würde, wenn die Probe 4 ausschließlich mittels des zweiten Lasers 18 bestrahlt würde. Durch eine zweite durchgezogene Linie 32 ist in Fig. 2 ein Temperaturverlauf dargestellt, der sich bei Kombination des ersten Lasers 12 (in Verbindung mit dem Raman-Konverter) mit dem zweiten Laser 18 und entsprechender Wahl der Leistung, des Zeitpunktes und der Zeitdauer der Laseremission ergibt.

Wie aus Fig. 2 ersichtlich ist, wird der zweite Laser hinsichtlich seiner Emission zu dem ersten Laser zeitverzögert angesteuert, wobei der Zeitpunkt und die Zeitdauer der Emission von Laserstrahlung durch den zweiten Laser 18 sowie dessen Leistung so gewählt sind, daß die Untersuchungstemperatur in der Probe 4 über einen bestimmten Zeitraum konstant oder im wesentlichen konstant bleibt.

## Patentansprüche

1. Einrichtung zur Untersuchung einer Probe (4) durch Erzeugung eines Temperatursprungs in der Probe (4),
mit einer Aufnahme (6) für die Probe (4),
mit Mitteln zur Erzeugung eines Temperatursprungs in der Probe (4) zur Erreichung einer Untersuchungstemperatur, die einen ersten Laser (12) aufweisen und
mit Mitteln zur Aufrechterhaltung der Untersuchungstemperatur, die wenigstens einen zweiten Laser (18) aufweisen.

2. Einrichtung zur Untersuchung einer Probe (4) durch Erzeugung eines Temperatursprungs in der Probe (4),
mit einer Aufnahme (6) für die Probe (4),
mit Mitteln zur Erzeugung eines Temperatursprungs in der Probe (4),
wobei die Mittel zur Erzeugung eines Temperatursprungs in der Probe (4) einen ersten Laser (12) und zweiten Laser (18) aufweisen und wobei Steuerungsmittel (22)
zur wahlweisen Ansteuerung der Laser vorgesehen sind.

3. Einrichtung nach Anspruch 1 oder 2, **dadurch gekennzeichnet, daß** der erste Laser (12) gepulste Laserstrahlung emittiert.

4. Einrichtung nach einem der vorhergehenden Ansprüche, **dadurch gekennzeichnet, daß** der zweite Laser (18) kontinuierlich Laserstrahlung emittiert.

5. Einrichtung nach einem der vorhergehenden Ansprüche, **dadurch gekennzeichnet, daß** der erste Laser (12) und/oder der zweite Laser (18) ein Infrarot-Laser sind bzw. ist.

6. Einrichtung nach einem der vorhergehenden Ansprüche, **dadurch gekennzeichnet, daß** der erste Laser (12) ein Nd-YAG-Laser ist.

7. Einrichtung nach einem der vorhergehenden Ansprüche, **dadurch gekennzeichnet, daß** der zweite Laser (18) ein Thulium-Laser ist.

8. Einrichtung nach einem der vorhergehenden Ansprüche, **dadurch gekennzeichnet, daß** dem ersten Laser (12) ein Frequenzwandler nachgeordnet ist.

9. Einrichtung nach Anspruch 8, **dadurch gekennzeichnet, daß** der Frequenzwandler ein Raman-Konverter (14) ist.

10. Einrichtung nach einem der vorhergehenden Ansprüche, **dadurch gekennzeichnet, daß** die Mittel zur Erzeugung eines Temperatursprungs in der Probe (4) und/oder die Mittel zur Aufrechterhaltung der Untersuchungstemperatur durch Steuerungs- und/oder Regelungsmittel (22) steuerbar bzw. regelbar sind.

11. Einrichtung nach Anspruch 10, **dadurch gekennzeichnet, daß** die Steuerungs- und/oder Regelungsmittel (22) elektronische Datenverarbeitungsmittel (24) aufweisen.

12. Einrichtung nach einem der Ansprüche 10 oder 11,
**dadurch gekennzeichnet, daß** die Steuerungs- und/oder Regelungsmittel (22) eine Detektoreinheit (26) zur Detektion der Probentemperatur aufweisen.
